# EUROPÄISCHE PATENTSCHRIFT

(11) **EP 4 110 242 B1**
(45) Veröffentlichungstag und Bekanntmachung des Hinweises auf die Patenterteilung: **18.02.2026**
(21) Anmeldenummer: 21710403.3
(22) Anmeldetag: 24.02.2021
(51) Int. Cl.: A61F 5/01

(54) **ORTHESENGELENK**
ORTHOSIS JOINT
ARTICULATION D'ORTHÈSE

(30) Priorität: 28.02.2020 DE 102020001327
(43) Veröffentlichungstag der Anmeldung: 04.01.2023
(73) Patentinhaber: Ottobock SE & Co. KGaA, 37115 Duderstadt (DE)
(72) Erfinder: SACHERER, Bernhard, 77948 Friesenheim (DE)
(74) Vertreter: Gramm, Lins & Partner Patent- und Rechtsanwälte PartGmbB
(86) Internationale Anmeldenummer: PCT/EP2021/025075
(87) Internationale Veröffentlichungsnummer: WO 2021/170296

(56) Entgegenhaltungen:
- EP-B1- 1 768 620
- US-A- 4 865 024
- US-A1- 2006 116 616
- US-A1- 2019 336 385
- US-B2- 7 192 407

## Beschreibung

Die Erfindung betrifft ein Orthesengelenk, insbesondere für ein Beingelenk, z.B. ein Hüft-, Knie- oder Knöchelgelenk, oder ein Armgelenk, wie ein Schulter-, Ellenbogen- oder ein Handgelenk, mit einem ersten Gelenkausleger und einem zweiten Gelenkausleger, die gegeneinander verschwenkbar miteinander verbunden sind, insbesondere mit beidseitiger Auslenkung, sowie mit wenigstens einem dabei zwischen den Gelenkauslegern als Rückstellelement wirkenden Funktionsmittel.

Ein solches Orthesengelenk ist insbesondere an Orthesen der unteren Extremitäten wie einer Knöchel-Fuß-Orthese AFO oder einer Knie-Knöchel-Fuß-Orthese KAFO vorteilhaft einsetzbar. Es ist jedoch nicht auf diesen Bereich oder auf diesen Einsatzzweck beschränkt und es kann theoretisch auch als Prothesengelenk verwendet werden.

Grundsätzlich ist es auch möglich ein derartiges Gelenk z.B. bei einer Lagerungstherapie anzuwenden, dies insbesondere auch im Bereich der oberen Extremitäten.

Die Bewegung in einem Körpergelenk kann mit Orthesengelenken unterstützt oder zum Schutz des Körpergelenks auch eingeschränkt werden, sofern dies aus therapeutischer Sicht sinnvoll ist. Bei der Bewegungseinschränkung wird durch das Orthesengelenk ein Anschlag für das Körpergelenk gebildet, wobei bis zum Anschlag ein möglichst sanft ansteigender Widerstand im Orthesengelenk aufgebaut werden soll, der dann auf das natürliche Körpergelenk einwirkt und die Auslenkung des Körpergelenkes begrenzt.

Für den Widerstand kann mit einem Orthesengelenk eine Rückstellkraft erzeugt werden, die das Körpergelenk in eine Ausgangsstellung zurückführt. In der Ausgangsstellung gibt es keine Rückstellkraft. Und bei einer Auslenkung des Körpergelenks aus dieser Ausgangsstellung heraus steigt die Rückstellkraft im Orthesengelenk vorzugsweise an. Die Ausgangsstellung wird auch als Grundstellung bezeichnet.

Bei einem Orthesengelenk, das mit einer Rückstellkraft arbeitet, werden die Gelenkausleger üblicherweise auf eine Grundstellung eingestellt, die der des Körpergelenks entspricht und bilden dabei einen Winkel zwischen dem ersten und dem zweiten Gelenkausleger.

Ein solches Orthesengelenk mit einer Grundstellung und einer Rückstellkraft bei beidseitigen Auslenkungen ist in der DE 20 2011 004 130 U1 offenbart. Diese Konstruktion verwendet als Funktionsmittel zwei seitlich an einem oberen Gelenkausleger angebrachte Tellerfederstapel, die in zwei Gehäusen untergebracht sind und die bei Auslenkung der Gelenkausleger eine starke Rückstellkraft erzeugen.

Für die Einstellung eines maximalen Funktionswinkels, um den die beiden Gelenkauslegern bei der Benutzung der Orthese gegeneinander aus der Grundstellung verschwenkt werden können, ist an jedem Tellerfederstapel ein Stellelement mit Anschlagsfläche vorgesehen, so dass der untere Gelenkausleger, der mit einer Fußbügelschale versehen ist, nur in einem begrenzten Winkelbereich gegenüber dem oberen Gelenkausleger verschwenkt werden kann.

Unterschiedliche Federeigenschaften werden durch austauschbare vorkonfigurierte Federmodule bereitgestellt. Eine Feinjustierung der maximalen Federkraft ist in einem geringen Umfang dabei durch Vorspannen der Tellerfederstapel möglich. Hierbei geht jedoch Federweg verloren und auch eine Minimalkraft werden ggf. erhöht.

Ansatzweise vergleichbare Konstruktionen finden sich in der DE 10 2016 107 779 A1 oder der DE 10 2013 011 382 A1 mit zwei federnden Funktionsmitteln beidseitig an einem oberen Gelenkausleger z.B. eines Knöchelgelenkes. Ein unterer Gelenkausleger ist z.B. eine Fußbügelschale, die an einem Fuß angeordnet werden kann. Je nach Richtung der in beide Richtungen möglichen Auslenkung wird eines der zwei Funktionsmittel angesteuert zur Erzeugung einer Rückstellkraft. Zum Einstellen der Rückstellkräfte sind hier die Funktionsmittel und ggf. die Gehäuse abnehmbar und auswechselbar. Für unterschiedliche Anforderungen steht dabei ein breites Sortiment an verschiedenen Funktionsmitteln bereit.

Problematisch bei allen vorbekannten Ausführungen bleibt, dass bei einem Verschwenken des Gelenkes aus der einen Richtung kommend über die Grundstellung hinaus in die andere Richtung beim Übergang bzw. während der Wechselphase der Auslenkungen in die entgegengesetzten Richtungen die von den Funktionsmitteln erzeugten Rückstellkräfte ruckartig abfallen und ansteigen. Genau in dieser Bewegungsphase kippt aber ein natürliches Gelenk nicht nur ohne Widerstand also ohne Rückstellkraft hin und her, sondern man hat auch jeweils sanft, also ruckfrei nachlassende und ansteigende Rückstellkräfte der Muskulatur und der Bänder.

Im Gegensatz hierzu erzeugen die Orthesengelenke nach dem Stand der Technik genau in dieser kritischen Bewegungsphase einen ruckartigen Übergang zwischen den in entgegengesetzten Richtungen wirkenden Funktionsmitteln.

Um einen derartigen ruckartigen Übergang zu vermeiden, kann für diese Bewegungsphase des Kippens des Orthesengelenkes ein Freilauf vorgesehen werden, bei dem keines der Funktionsmittel eine Kraft ausübt. Der Kraftschluss wird damit aber sprunghaft unterbrochen, was damit einhergeht, dass dann beim Wechsel der Beaufschlagung der Funktionsmittel Geräusche auftreten, mit denen auch Verschleiß verbunden ist.

Auch den kosmetischen Ansprüchen werden derartige bekannte Orthesengelenke z.B. als Knöchelgelenke aufgrund der zwei ausladenden Funktionsmittel nur bedingt gerecht.

US 2006/116616 offenbart ein Orthesengelenk gemäß dem Oberbegriff des Anspruchs 1.

Aufgabe der vorliegenden Erfindung ist es deshalb, ein gegenüber dem Stand der Technik kompakteres und leichteres Orthesengelenk bereit zu stellen, das mit umfangreichen Einstellmöglichkeiten eines Funktionswinkels und mit einer physiologisch eingeleiteten Rückstellkraft eine natürlich anmutende Bewegung ermöglicht.

Erfindungsgemäß wird diese Aufgabe durch ein Gelenk mit den Merkmalen des Hauptanspruchs gelöst. Vorteilhafte Ausgestaltungen und Weiterbildungen der Erfindung sind in den Unteransprüchen, der Beschreibung sowie den Figuren offenbart.

Das erfindungsgemäße Orthesengelenk sieht vor, die Rückstellkraft mit nur einem Funktionsmittel zu erzeugen, das aus wenigstens einer Biegefeder besteht, die im Wesentlichen längs ausgerichtet ist. Durch die Verwendung nur einer Biegefeder kann das Orthesengelenk deutlich kleiner ausgestaltet werden als die bisher bekannten Ausführungsformen.

Bei einer bevorzugten Ausführungsform der Erfindung ist die Biegefeder als Blattfeder ausgestaltet, wobei bei einer besonders bevorzugten Ausführungsform diese Blattfeder eine Stapelanordnung aufweist. Unter einer derartigen Stapelanordnung wird im Rahmen dieser Erfindung verstanden, eine Mehrzahl von Einzelblattfedern, die z.B. unterschiedliche Längen haben, nebeneinander anzuordnen, d.h. zu stapeln, so dass die Einzelblattfedern je nach Auslenkung erst sukzessive zum Einsatz kommen.

Durch eine derartige Stapelanordnung kann die Blattfeder innerhalb des Orthesengelenkes auch bedarfsweise umgestapelt werden, wodurch das Orthesengelenk bedarfsweise angepasst oder konfiguriert werden kann. Ein zusätzliches Sortiment an Spezialteilen ist nicht erforderlich. Dafür ist das erfindungsgemäße Orthesengelenk mit einer variablen Aufhängung bzw. Lagerung der Einzelblattfedern ausgestattet.

Die Anwendung von Biegefedern in Orthesengelenken ist grundsätzlich zwar bekannt, sie werden aber bisher nur für andere Zwecke eingesetzt. So ist z.B. aus der EP 1 768 620 B1 ein polyzentrisches Orthesengelenk bekannt, das dafür bestimmt ist, einen einseitigen Streckanschlag für die Kniestreckung zu erzeugen. Dafür werden verschiedene Abstandhalter zwischen Anschlagflächen eingefügt, wobei diese Abstandhalter über Blattfedern in Position gehalten werden. Eine federnde Rückstellkraft, die auf die beiden Gelenkausleger oder auf die Gliedmaßen wirksam ist, ist dabei nicht offenbart.

Für die Erzeugung der Rückstellkräfte sind bei dem erfindungsgemäßen Orthesengelenk die Ansteuerungspunkte seitlich des Funktionsmittels, also der Biegefeder bzw. Blattfeder bzw. Blattfederanordnung in einer Dreiecksanordnung angelegt. Bei der Auslenkung der Gelenkausleger in eine erste Richtung werden wenigstens drei Ansteuerungspunkte, die an den Gelenkauslegern in Form von Übertragungselementen zu finden sind, quer zu der Längsachse der Biegefeder bzw. Blattfeder bzw. Blattfederanordnung verschoben, wodurch das Funktionselement verbogen wird und somit eine Rückstellkraft erzeugt.

Dieses bekannte Prinzip einer Dreiecksanordnung wird bei dem erfindungsgemäßen Gelenk grundsätzlich auch noch einmal gespiegelt angewendet. Dabei können durch unterschiedliche Abstände der Ansteuerungspunkte auf den beiden Seiten des Funktionsmittels die Rückstellkräfte unterschiedlich starke Anstiege haben.

Ein Funktionsmittel kann entweder parallel zu einer Längsachse des Orthesengelenkes liegen, die im Wesentlichen durch eine mit der Orthese zu versehende Extremität in gestrecktem Zustand definiert wird, oder senkrecht zu dieser Längsachse angeordnet werden. Die Übertragungselemente können an einer Seite der Blattfederanordnung angeordnet sein. Und si können bzgl. ihrer Abstände zusammengeschoben werden. Die erzeugten Rückstellkräfte der Biegefeder gehen bei jeder Variante bei abwechselnder Auslenkung ruckfrei in einander über.

Die Gelenkausleger am Orthesengelenk stehen dabei in ständigem Kraftschluss mit dem Funktionselement, so dass aufgrund der Rückstellkraft kein ruckartiger Übergang mehr stattfindet. Der mit einem erfindungsgemäßen Gelenk mögliche Bewegungsablauf ist damit vergleichbar bei einem natürlichen Gelenk mit dem ruckfreien und ständigen Kraftschuss der Muskulatur.

In der Grundstellung befindet sich das Funktionsmittel, also die Biegefeder bzw. die Blattfeder oder Blattfederanordnung in einer Ruhelage, die ohne innere Spannung ist. In dieser Ruhelage ist es ein Leichtes, die verstellbar auf den Gelenkauslegern angeordneten Übertragungselemente entlang deren Längsachse stufenlos zu verschieben und dann zu fixieren. So kann der Abstand der Ansteuerungspunkte an dem Funktionsmittel zueinander verändert werden, womit die Biegelänge und in diesem Zusammenhang die Steifigkeit des Funktionsmittels, und damit die Rückstellkraft der Biegefeder, der Blattfeder bzw. Blattfederanordnung verändert wird.

Bei einer besonders bevorzugten Ausführungsform der Erfindung weist einer der Gelenkausleger, insbesondere der zweite untere Gelenkausleger, einen Anlenkarm auf, zu dem er in einer beliebigen Winkelstellung stufenlos verdreht und arretiert werden kann. Die Krafteinleitung vom Funktionsmittel auf den zweiten Gelenkausleger erfolgt dann über diesen Anlenkarm. Insbesondere wird vorgeschlagen, den Anlenkarm und den zweiten Gelenkausleger auf einem gemeinsamen Drehpunkt zu lagern, um einen belastbaren Gegenhalt zur Klemmung zu erzeugen.

Eine andere Variante sieht hierfür vor, dass zwischen dem Anlenkarm und dem unteren Gelenkausleger ein zusätzlicher Riegel wirksam ist, der in wenigstens eine Nut an dem Anlenkarm eingreift, um einen oder mehrere vorgegebene Funktionswinkel zwischen dem ersten und zweiten Gelenkausleger einzustellen.

Weitere zweckmäßige und/oder vorteilhafte Merkmale der Erfindung ergeben sich aus den Unteransprüchen und der nachfolgenden Beschreibung der Zeichnung. Besonders bevorzugte Ausführungsformen der Erfindung werden hierzu anhand der beigefügten Zeichnung näher erläutert. Dabei zeigt:
- Fig. 1: die Ansicht einer beispielhaften Biege- und einer Blattfeder,
- Fig. 2: die Vorderansicht einer Anordnung mit Blattfedern,
- Fig. 3: die Vorderansicht einer einseitigen Dreiecksanordnung der Ansteuerungspunkte mit beidseitiger Wirkung.
- Fig. 4: die Vorderansicht einer alternativen einseitigen Dreiecksanordnung der Ansteuerungspunkte mit beidseitiger Wirkung,
- Fig. 5: die Vorderansicht einer beidseitigen Dreiecksanordnung der Ansteuerungspunkte mit beidseitiger Wirkung,
- Fig. 6: die Vorderansicht eines Orthesengelenkes in Ruhelage,
- Fig. 7: die Vorderansicht eines Orthesengelenkes gemäß Fig. 6 in linksseitiger Auslenkung,
- Fig. 8: die Vorderansicht einer alternativen Ausführungsform eines Orthesengelenkes z.B. als Knöchelgelenk in linksseitiger Auslenkung.

In den Zeichnungen sind die gleichen Teile im Wesentlichen mit den gleichen Bezugsziffern versehen.

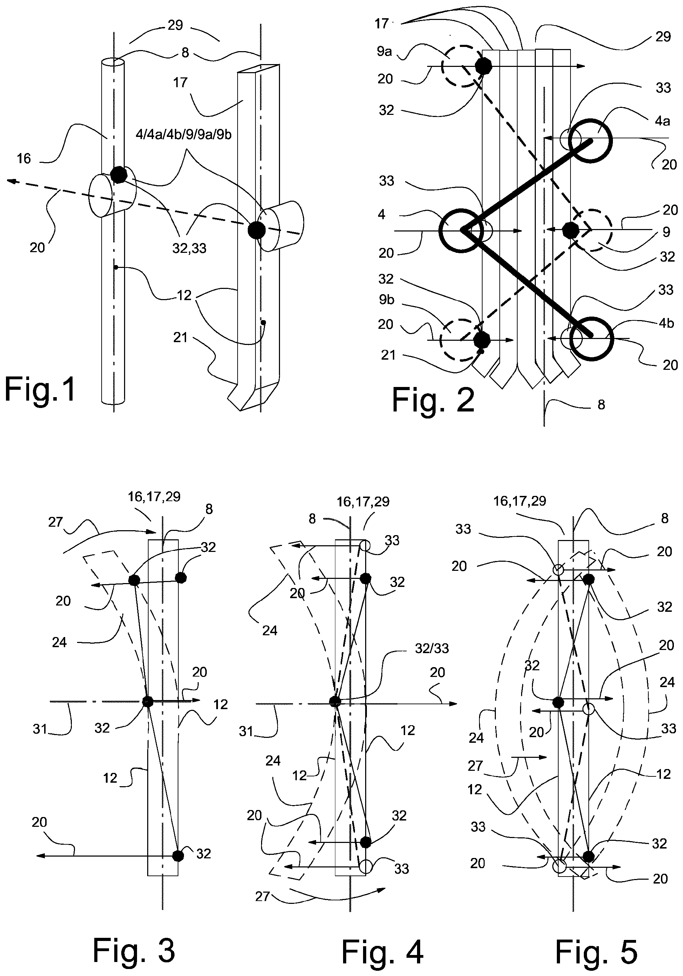

Die Fig. 1 zeigt zwei Ausführungsformen von erfindungsgemäßen Funktionsmitteln 29 in der Form als Biegefeder 16 oder als Blattfeder 17, die hier in einer gerade ausgerichteten Form dargestellt sind, wobei die Blattfeder 17 an einem Ende mit einer stirnseitigen Abwinkelung 21 versehen ist. Mit dieser stirnseitigen Abwinkelung 21 kann eine einzelne Feder in einer Vorrichtung aufgehängt sein, um das Abgleiten einer Blattfeder 17 zu verhindern. Erkennbar sind auch Übertragungselemente 4, 4a, 4b, 9, 9a, 9b und die Richtung einer Krafteinleitungsline 20, durch die Ansteuerungspunkte 32, 33 an einer insbesondere seitlich außen am Funktionsmittel 29 befindlichen Übertragungsfläche 12 gebildet werden.

Fig. 2 zeigt eine schematische Vorderansicht eines Funktionsmittels 29 für ein Orthesengelenk 5 in der Form einer Blattfederanordnung, bestehend aus mehreren nebeneinander angeordneten, gestapelten Blattfedern 17.

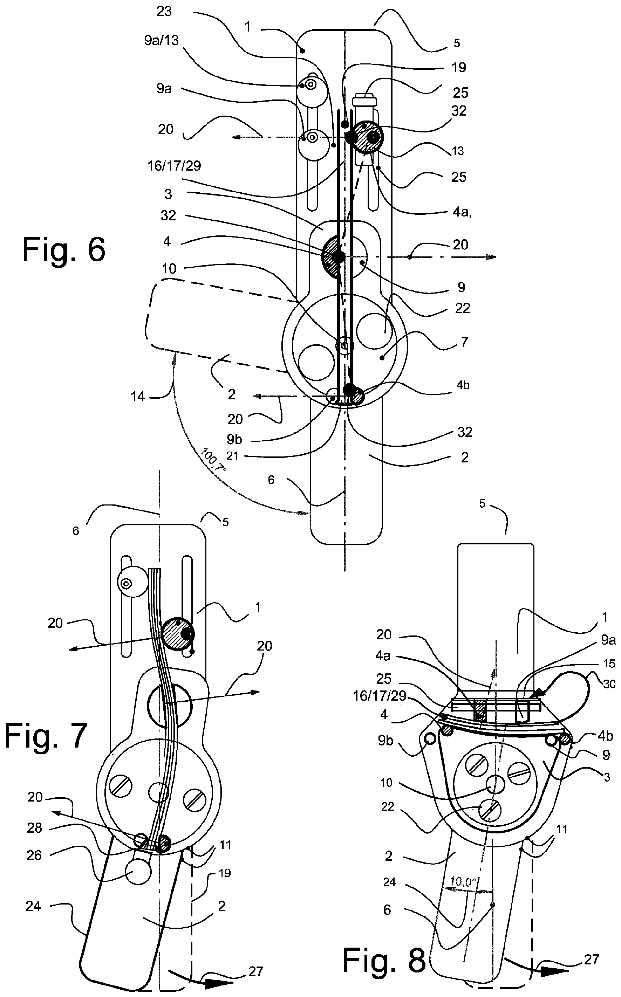

Das Funktionsmittel 29 ist hier schematisch in Form einer bevorzugten Anordnung von Blattfedern 17 dargestellt und zwischen den Übertragungselementen 4, 4a, 4b, 9, 9a, 9b gelagert. Bei einer Auslenkung 24 von Gelenkauslegern 1, 2, auf denen die Übertragungselementen 4, 4a, 4b, 9, 9a, 9b sitzen, werden Kräfte auf das Funktionsmittel 29 geleitet, die mit den Krafteinleitungslinien 20 quer zu einer Längsachse 8 der Blattfedern 17 dargestellt sind. Die Übertragungselemente 4, 4a, 4b bzw. die Übertragungselemente 9, 9a, 9b sind jeweils in einer Dreiecksanordnung mit Verbindungslinien dargestellt. Daraus ergeben sich auch die Ansteuerungspunkte 32, 33 die alternativ bei Auslenkungen 24 in entgegengesetzten Richtungen wirksam sind. Bei entsprechenden Auslenkungen 24 der Gelenkausleger 1, 2 werden jeweils Rückstellkräfte 27 an den Gelenkauslegern 1, 2 erzeugt, wie weiter unten anhand der Fig. 7, 8 erläutert wird.

Fig. 3 zeigt schematisch eine Blattfeder 17 mit nur einer Dreiecksanordnung der Ansteuerungspunkte 32 zur Erzeugung einer Rückstellkraft 27. Bei dieser Variante werden nur drei Ansteuerungspunkte 32 um die Spiegelachse 31 auf der Krafteinleitungslinie 20 gespiegelt. Dabei werden die Enden der Blattfeder 17 abwechselnd angesteuert. Der Vorteil hierbei ist die sehr kompakte Bauweise. Die Rückstellkraft 27 der Blattfeder 17 kann durch Verschieben des mittleren Ansteuerungspunktes 32 parallel zur Längsachse der Blattfeder 17 zu jeder Seite verändert werden.

Fig. 4 zeigt eine besonders bevorzugte Variante mit einem Funktionsmittel 29 mit zwei Dreiecksanordnungen, die mit wenigstens fünf Ansteuerungspunkten 32, 33 gebildet werden zur Erzeugung einer Rückstellkraft 27. Bei dieser Variante sind die Ansteuerungspunkte 32, 33 auf der Spiegelachse 31 der Krafteinleitungslinie 20 gespiegelt. Dabei werden die Enden der Blattfeder 17 abwechselnd ausgelenkt, wodurch jeweils zwei Ansteuerungspunkte 32, 33 in der Dreiecksanordnung abwechselnd wirksam sind.

Fig. 5 zeigt schematisch zwei auf der Längsachse 8 gespiegelte Dreiecksanordnungen der Ansteuerungspunkte 32, 33 an einer Blattfeder 17. Man erkennt dabei die Kraft auf der Krafteinleitungslinie 20 zur Erzeugung einer Verbiegung und einer Rückstellkraft 27. Ein Merkmal ist die Spiegelung von sechs Ansteuerungspunkten 32, 33 in zwei Dreiecksanordnungen auf der Längsachse 8. Die alternativ in beide Richtungen erzeugte Rückstellkraft 27 kann durch Verschieben eines beliebigen Ansteuerungspunktes verändert werden.

Die Fig. 6 zeigt eine schematische Vorderansicht eines Orthesengelenkes 5. Man erkennt zwei auf der Längsachse 8 gespiegelte Dreiecksanordnungen der Ansteuerungspunkte 32, 33 an einer Blattfeder 17 gemäß der Fig. 5.

Das Funktionsmittel 29 zur Erzeugung einer Rückstellkraft 27 ist hier im Bereich eines Drehpunkt 10 des Orthesengelenkes 5 angeordnet. Gut zu erkennen sind die Gelenkausleger 1, 2, die am Drehpunkt 10 miteinander verbunden sind. Beide Gelenkausleger 1, 2 sind zueinander nach rechts und links verschwenkbar. Das Funktionsmittel befindet sich bei dieser Darstellung in der Ruhelage 19, also ohne Auslenkung der Gelenkausleger.

Der untere Gelenkausleger 2 ist auf dem Drehpunkt 10 zusammen mit einem Anlenkarm 3 gelagert zum Einstellen eines abgebildeten Funktionswinkels 14 zwischen Gelenkausleger 1 und dem Gelenkausleger 2. Wie man erkennt, bleibt der Winkel zwischen dem Gelenkausleger 1 und dem Anlenkarm 3 immer unverändert, selbst wenn die Gelenkausleger 1 und 2 gegeneinander verschwenkt werden.

Der Anlenkarm 3 kann zu dem Gelenkausleger 2 nach beiden Seiten der Mittellinie stufenlos verschwenkt werden und mit einem Spannmittel 22 gegenüber dem Gelenkarm 2 arretiert werden. Der zweite Gelenkausleger 2 bleibt so mit jedem eingestellten Funktionswinkel 14 über den Anlenkarm 3 im Kraftschluss mit dem Funktionsmittel 29 und mit dem ersten Gelenkausleger 1 verbunden.

Für jede der o.g. Dreiecksanordnungen sind Übertragungselemente 4, 4a, 4b, 9, 9a, 9b auf dem ersten Gelenkausleger 1 mit Verschiebemitteln 25 entlang der Längsachse 8 der Biegefeder 16 bzw. Blattfeder 17 verstellbar und arretierbar angeordnet. Damit kann die Rückstellkraft 27 des Funktionsmittels 29 verändert werden.

Ein außerhalb der Reichweite des Funktionsmittels 29 positioniertes Übertragungselement 9/23 ermöglicht dabei auch eine freie Auslenkung der Gelenkausleger 1, 2 und setzt die Funktionsstellung und die Rückstellkraft 27 außer Funktion.

An dem Übertragungselement 9a ist eine als Exzenter 13 angelegte Welle erkennbar. Beim Verdrehen des Übertragungselements 9a um den Exzenter 13 kann ein begrenzter Freilauf 23 des Übertragungselementes zum Funktionsmittel 29 eingestellt werden, bei dem der Gelenkausleger 1 von der Rückstellkraft 27 entkoppelt ist.

Fig. 7 zeigt eine Vorderansicht einer Ausführungsform des Orthesengelenkes 5, dargestellt mit einer linksseitigen Auslenkung 24 der Gelenkausleger 1, 2, wobei gleichzeitig der Anlenkarm 3 nach rechts ausgelenkt ist zum Bilden einer Rückstellkraft 27 an der Blattfeder 17.

Das Prinzip mit zweifacher Dreiecksanordnung der Ansteuerungspunkte 32, 33 ist hier gespiegelt um die Längsachse 8 des Funktionsmittels 29 angewendet.

Die dargestellte Anordnung von mehreren Blattfedern 17, die zu einem Blattfederstapel zusammengefasst sind, ist für eine beidseitige Auslenkung zwischen sechs Übertragungselementen 4, 4a, 4b und 9, 9a, 9b eingespannt gemäß Fig. 2, 5. Bei der dargestellten linksseitigen Auslenkung 24 des Anlenkarmes 3 werden z.B. die einzelnen Blattfedern 17 über den Anlenkarm 3 gebogen.

Beim gemeinsamen Auslenken des Gelenkauslegers 2 und des Anlenkarmes 3 stellt sich bei dem hier dargestellten Ausführungsbeispiel ein drehbar gelagertes Übertragungselement 4, 9 auf den Verlauf der Blattfeder 17 bzw. des durch diese gebildeten Blattfederstapels ein.

Durch eine Veränderung des Abstandes zwischen den Übertragungselementen 4a, 9a zu den Übertragungselementen 4, 9 wird die aktive Biegelänge der hier dargestellten Anordnung von Blattfedern 17 verändert und erzeugt somit einen unterschiedlichen Verlauf der Biegung zwischen den Übertragungselementen 4, 9 und 4a, 9a mit einer daraus resultierenden unterschiedlichen Rückstellkraft 27.

Für die Einstellung eines Funktionswinkels 14 werden die Spannmittel 22, gelöst, sodass der zweite Gelenkausleger 2 in Relation zum Anlenkarm 3 verdreht werden kann. Über die Klemmflächen 11 zwischen der Welle 7 und dem Anlenkarm 3 ist der zweite Gelenkausleger 2 in der gewählten Stellung stufenlos zu arretieren.

Eine vorbestimmte Einstellung eines Funktionswinkels 14 ist mit einem Riegel 26 und wenigstens einer Nut 28 am Anklenkarm 3 möglich. Bei einer einfachen Ausführung des Gelenks 5 mit eingeschränkter Verstellbarkeit des Funktionswinkels 14 sind so die Übertragungselemente 4b, 9b und die Übertragungselemente 4, 9 direkt auf dem zweiten Gelenkausleger 2 angeordnet. Damit ist der Anlenkarm 3 nicht verdrehbar gegenüber dem zweiten Gelenkausleger 2 und ein Funktionswinkel 14 ist nur eingeschränkt einstellbar. Die beidseitige Rückstellkraft 27 bleibt dabei erhalten.

Fig. 8 zeigt eine alternative Ausführung eines Orthesengelenkes 5 als Knöchelgelenk mit linksseitiger aktiver Auslenkung 24 der Gelenkausleger 1, 2 und des Anlenkarmes 3 zum Bilden einer Rückstellkraft 27 an der Blattfeder 17. Das Prinzip ist hier mit einer Dreiecksanordnung der Ansteuerungspunkte 32, 33 gemäß der Fig. 4 angewendet. Dabei sind die Übertragungselemente 4, 4b und 9, 9b auf dem ersten Gelenkausleger 1 angeordnet. Bei der dargestellten linksseitigen Auslenkung 24 des Anlenkarmes 3 werden die einzelnen Blattfedern 17 an einem Ende über den Anlenkarm 3 gebogen.

Bei dieser Ausführung des Gelenkes 5 können die einzelnen Blattfedern 17 innerhalb des Gelenkes 5 an die Außenkante der Übertragungselemente 4, 9, gemäß dem Pfeil 30 umpositioniert werden, wobei die Übertragungselemente 4, 9 zwischen den Blattfedern 17 in Position gehalten werden. Diese bilden damit konfigurierbare Blattfedern 15. Damit ist es möglich, die Rückstellkraft 27 dieser Blattfedern 15 für jedes Körpergewicht zu konfigurieren. Feineinstellungen bezüglich der Rückstellkraft 27 werden durch Verschieben der Übertragungselemente 4, 9 entlang der Blattfeder 15 vorgenommen.

Die Erfindung bietet vielseitige Möglichkeiten und ist nicht auf die bildlich dargestellten Ausführungsformen an Orthesengelenken 5 beschränkt.

### Bezugszeichenliste

- 1.: erster, oberer Gelenkausleger
- 2.: zweiter, unterer Gelenkausleger
- 3.: Anlenkarm
- 4, 4a, 4b: Übertragungselement
- 5: Orthesengelenk
- 6: Mittellinie Gelenk
- 7: Welle
- 8: Längsachse des Funktionsmittels
- 9, 9a,9b: Übertragungselement
- 10.: Drehpunkt
- 11.: Klemmflächen
- 12.: Übertragungsfläche am Funktionsmittel
- 13.: Exzenter
- 14.: Funktionswinkel
- 15.: Konfigurierbare Blattfeder
- 16.: Biegefeder
- 17.: Blattfeder
- 20.: Krafteinleitungslinie
- 21.: Abwinkelung einer Blattfeder
- 22.: Spannmittel
- 23.: Freilauf
- 24.: Auslenkung mit Rückstellkraft
- 25.: Verschiebemittel
- 26.: Riegel
- 27.: Rückstellkraft
- 28.: Nut
- 29.: Funktionsmittel
- 30.: Pfeil
- 31.: Spiegelachse
- 32.: Ansteuerungspunkt Dreiecksanordnung
- 33.: Ansteuerungspunkt Dreiecksanordnung

## Patentansprüche

1. Orthesengelenk, mit einem ersten Gelenkausleger (1) und einem zweiten Gelenkausleger (2), die gegeneinander verschwenkbar gelagert sind, und wenigstens einem zwischen den Gelenksauslegern (1, 2) als Rückstellelement wirkenden Funktionsmittel (29),
**dadurch gekennzeichnet,**
**dass** das Funktionsmittel (29) wenigstens eine Biegefeder (16, 17) aufweist, die beidseitig bei einer Auslenkung der Gelenkausleger (1, 2) eine Rückstellkraft (27) erzeugt.

2. Orthesengelenk nach Anspruch 1,
**dadurch gekennzeichnet,**
**dass** das Funktionsmittel (29) eine Blattfeder (17) mit Stapelanordnung aufweist.

3. Orthesengelenk nach einem oder mehreren der vorstehenden Ansprüche,
**dadurch gekennzeichnet,**
**dass** an dem Funktionsmittel (16, 17, 29) wenigstens drei Ansteuerungspunkte (32) in einer Dreiecksanordnung angeordnet sind zur Erzeugung von in entgegengesetzten Richtungen unterschiedlichen Rückstellkräften (27).

4. Orthesengelenk nach einem oder mehreren der vorstehenden Ansprüche,
**dadurch gekennzeichnet,**
**dass** wenigstens ein Ansteuerungspunkt (32, 33) über ein Übertragungselement (4, 4a, 4b, 9, 9a, 9b) an einem der Gelenkausleger (1, 2) verstellbar angeordnet ist zur Veränderung der Rückstellkraft (27) des Funktionsmittels (16, 17, 29).

5. Orthesengelenk nach einem oder mehreren der vorstehenden Ansprüche,
**dadurch gekennzeichnet,**
**dass** die Gelenkausleger (1, 2) für einen Freilauf (23) entkoppelbar sind.

6. Orthesengelenk nach einem oder mehreren der vorstehenden Ansprüche,
**dadurch gekennzeichnet,**
**dass** ein Anlenkarm (3) verstellbar und arretierbar mit dem zweiten Gelenkausleger (2)verbunden ist zur Einstellung eines Funktionswinkels (14) zwischen den Gelenkauslegern (1, 2).

7. Orthesengelenk nach Anspruch 6
**dadurch gekennzeichnet,**
**dass** der zweite Gelenkausleger (2) mit dem Anlenkarm (3) über gemeinsame Klemmflächen (11) stufenlos arretierbar ist.

8. Orthesengelenk nach Anspruch 6,
**dadurch gekennzeichnet,**
**dass** der Anlenkarm (3) mit wenigstens einer Nut (28) versehen ist, zum Arretieren in einem Funktionswinkel (14) gegenüber dem zweiten Gelenkausleger (2).

9. Orthesengelenk nach einem oder mehreren der Ansprüche 6 und 7 oder 8,
**dadurch gekennzeichnet,**
**dass** die Krafteinleitung von dem Funktionsmittel (29) auf den zweiten Gelenkausleger (2) nur über den Anlenkarm (3) erfolgt.

10. Orthesengelenk nach einem oder mehreren der Ansprüche 6 bis 9,
**dadurch gekennzeichnet,**
**dass** der Anlenkarm (3) mit dem ersten Gelenkausleger (1) und dem zweiten Gelenkausleger (2) einen gemeinsamen Drehpunkt (10) aufweist.

## Claims

1. An orthosis joint with a first joint arm (1) and a second joint arm (2), which are mounted such that they can pivot with respect to one another, and at least one functional element (29) acting between the joint arms (1, 2) as a restoring element,
**characterized in that**
the functional element (29) has at least one bending spring (16, 17) which generates a restoring force (27) on one side or the other when the joint arms (1, 2) are deflected.

2. The orthosis joint according to claim 1,
**characterized in that**
the functional element (29) has a leaf spring (17) with a stacking arrangement.

3. The orthosis joint according to one or more of the preceding claims,
**characterized in that**
at least three actuation points (32) are arranged in a triangular arrangement on the functional element (16, 17, 29) for generating different restoring forces (27) in opposite directions.

4. The orthosis joint according to one or more of the preceding claims,
**characterized in that**
at least one actuation point (32, 33) is adjustably arranged via a transmission element (4, 4a, 4b, 9, 9a, 9b) on one of the joint arms (1, 2) for changing the restoring force (27) of the functional element (16, 17, 29).

5. The orthosis joint according to one or more of the preceding claims,
**characterized in that**
the joint arms (1, 2) can be decoupled for a freewheel (23).

6. The orthosis joint according to one or more of the preceding claims,
**characterized in that**
an articulated arm (3) is connected to the second joint arm (2) in an adjustable and lockable manner for setting a functional angle (14) between the joint arms (1, 2).

7. The orthosis joint according to claim 6,
**characterized in that**
the second joint arm (2) can be continuously locked with the articulated arm (3) via common clamping surfaces (11).

8. The orthosis joint according to claim 6,
**characterized in that**
the articulation arm (3) is provided with at least one groove (28) for locking at a functional angle (14) relative to the second joint arm (2).

9. The orthosis joint according to one or more of claims 6 and 7 or 8,
**characterized in that**
the force transmission from the functional element (29) to the second joint arm (2) takes place only via the articulated arm (3).

10. The orthosis joint according to one or more of claims 6 to 9,
**characterized in that**
the articulated arm (3) has a common pivot point (10) with the first joint arm (1) and the second joint arm (2).

## Revendications

1. Articulation d'orthèse, comprenant un premier bras articulé (1) et un deuxième bras articulé (2) qui sont montés de manière à pouvoir pivoter l'un par rapport à l'autre, et comprenant au moins un moyen fonctionnel (29) agissant comme élément de rappel entre les bras articulés (1, 2),
**caractérisée en ce que**
le moyen fonctionnel (29) comporte au moins un ressort de flexion (16, 17) qui génère une force de rappel (27) des deux côtés lors d'un débattement des bras articulés (1, 2).

2. Articulation d'orthèse selon la revendication 1,
**caractérisée en ce que**
le moyen fonctionnel (29) comprend un ressort à lame (17) avec un agencement empilé.

3. Articulation d'orthèse selon l'une ou plusieurs des revendications précédentes,
**caractérisée en ce que**
au moins trois points de commande (32) sont disposés en triangle sur le moyen fonctionnel (16, 17, 29) afin de générer des forces de rappel (27) différentes dans des directions opposées.

4. Articulation d'orthèse selon l'une ou plusieurs des revendications précédentes,
**caractérisée en ce que**
au moins un point de commande (32, 33) est disposé de manière réglable sur l'un des bras articulés (1, 2) via un élément de transmission (4, 4a, 4b, 9, 9a, 9b) afin de modifier la force de rappel (27) du moyen fonctionnel (16, 17, 29).

5. Articulation d'orthèse selon l'une ou plusieurs des revendications précédentes,
**caractérisée en ce que**
les bras articulés (1, 2) peuvent être désaccouplés pour permettre une course à roue libre (23).

6. Articulation d'orthèse selon l'une ou plusieurs des revendications précédentes,
**caractérisée en ce que**
un levier de liaison (3) est relié de manière réglable et verrouillable au deuxième bras articulé (2) afin de régler un angle fonctionnel (14) entre les bras articulés (1, 2).

7. Articulation d'orthèse selon la revendication 6,
**caractérisée en ce que**
le deuxième bras articulé (2) muni du levier de liaison (3) peut être verrouillé en continu via des surfaces de serrage communes (11).

8. Articulation d'orthèse selon la revendication 6,
**caractérisée en ce que**
le levier de liaison (3) est pourvu d'au moins une rainure (28) pour le verrouillage selon un angle fonctionnel (14) par rapport au deuxième bras articulé (2).

9. Articulation d'orthèse selon l'une ou plusieurs des revendications 6 et 7 ou 8,
**caractérisée en ce que**
la transmission de la force du moyen fonctionnel (29) au deuxième bras articulé (2) s'effectue uniquement via le levier de liaison (3).

10. Articulation d'orthèse selon l'une ou plusieurs des revendications 6 à 9,
**caractérisée en ce que**
le levier de liaison (3) présente un point de rotation (10) en commun avec le premier bras articulé (1) et le deuxième bras articulé (2).
